# FASCICULE DE BREVET EUROPEEN

(11) **EP 1 144 428 B1**
(45) Date de publication et mention de la délivrance du brevet: **13.10.2004**
(21) Numéro de dépôt: 00900224.7
(22) Date de dépôt: 17.01.2000
(51) Int. Cl.: C07H 17/08, A61K 31/70

(54) **PROCEDE POUR PREPARER UN AZA-MARCROLIDE AVEC 4''(R) - NH2**
VERFAHREN ZUR HERSTELLUNG VON AZA-MAKROLIDEN MIT 4"(R)-NH2
METHOD FOR PREPARING AN AZA-MACROLIDE WITH 4"(R)-NH2

(30) Priorité: 18.01.1999 FR 9900459
(43) Date de publication de la demande: 17.10.2001
(73) Titulaire: MERIAL, 69002 Lyon (FR)
(72) Inventeur: LEON, Patrick, F-69160 Tassin-la-Demi-Lune (FR); LHERMITTE, Frédéric, F-69360 Saint Symphorien D'Ozon (FR); GUEVEL, Ronan, F-69008 Lyon (FR); PAUZE, Denis, F-69360 Solaize (FR); GAREL, Laurent, F-69003 Lyon (FR); ODDON, Gilles, F-69003 Lyon (FR)
(74) Mandataire: Nargolwalla, Cyra
(86) Numéro de dépôt international: PCT/FR2000/000089
(87) Numéro de publication internationale: WO 2000/042056

(56) Documents cités:
- EP-A- 0 508 699
- EP-A- 0 549 040
- WO-A-99/12542
- K.SHANKARAN ET AL.: "Preparation and Activities of 4"-Epi and 4"-deoxy-4"-Amino Analogs Derived From 9-Deoxo-8a-Aza-8a-Homoerythromycin A." BIOORGANIC AND MEDICINAL CHEMISTRY LETTERS, vol. 4, no. 9, 1994, pages 1111-1116, XP002118137

## Description

La présente invention a pour objet un procédé notamment utile pour transformer la fonction 4"(S)-OH du motif cladinose d'un aza-macrolide en 4"(R)-NH₂.

La présente invention concerne plus particulièrement le domaine des antibiotiques macrotides de type érythromycine et plus particulièrement leurs dérivés aza-macrolides qui font l'objet du brevet EP 508 699 et répondent à la formule générale suivante : 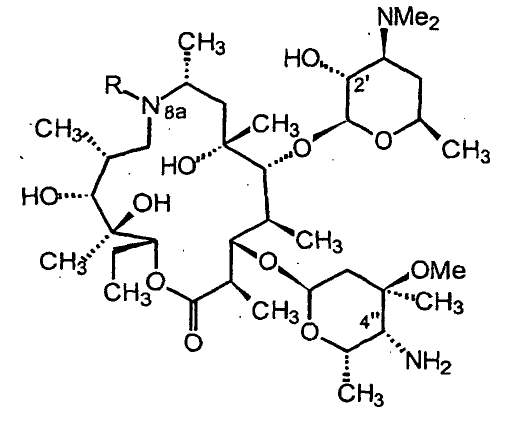 dans laquelle R représente un atome d'hydrogène, un groupement alkyle en C₁-C₁₀, alkényle en C₂C₁₀ ou arylesulfonyle en C₆-C₁₂, le cas échéant substitués.

La publication *Bioorganic & Medicinal Chemistry Letters,* vol. 4, n°9, pp. 1111-1115, 1994, décrit également la synthèse et l'activité biologique de dérivés 9-déoxo-8a-aza-8a-homoérythromycine A répondant à la formule générale précédente, diversement substitués en position 8a et 4"-amino.

Ces composés sont obtenus à partir de l'érythromycine et leur synthèse implique deux étapes majeures :
- la création du macrocycle 8a-azalide au départ de l'oxime (Z) qui subit un réarrangement stéréospécifique de Beckmann et
- la modification du groupe cladinose en position 4" consistant en la transformation du 4"(S)-OH en 4"(R)-NH₂ c'est-à-dire avec inversion de configuration que l'on peut illustrer comme suit :

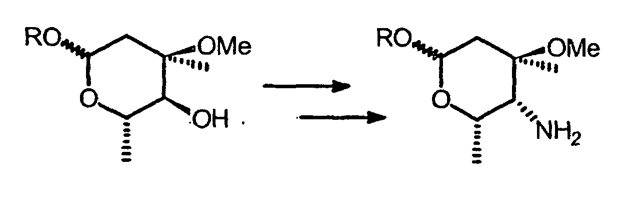

En fait, la voie actuellement retenue pour assurer cette transformation de la fonction 4"(S)-OH en 4"(R)-NH₂ n'est pas totalement appropriée à une production à l'échelle industrielle.

Elle implique successivement une oxydation de la fonction hydroxyle en position 4" en fonction cétonique puis la transformation de cette cétone en oxime qui par réduction conduit à un mélange d'environ 1 pour 1 du dérivé amino attendu et de son épimère en 4".

Cette voie de synthèse a par conséquent pour inconvénient majeur de requérir la formation d'intermédiaires C-4" sp₂ et donc de perdre l'information stéréochimique présente initialement au niveau du C-4" sp₃ du motif cladinose. Ce résultat est d'autant plus gênant que les isomères, acquis à l'issue de cette voie de synthèse, sont obtenus avec un rendement faible de l'ordre de 20 % et sont en outre difficilement séparables. C'est ainsi que pour une rendement brut de réaction de l'ordre de 20 % on obtient seulement environ 7 % du dérivé amino avec inversion de configuration.

La présente invention a précisément pour objet de proposer une nouvelle voie d'accès à ces dérivés aminés en position 4" qui avantageusement permet de préserver une stéréosélectivité significative et procure un rendement satisfaisant.

Plus précisément, la présente invention a pour premier objet un procédé de préparation stéréosélectif d'un composé de formule générale I 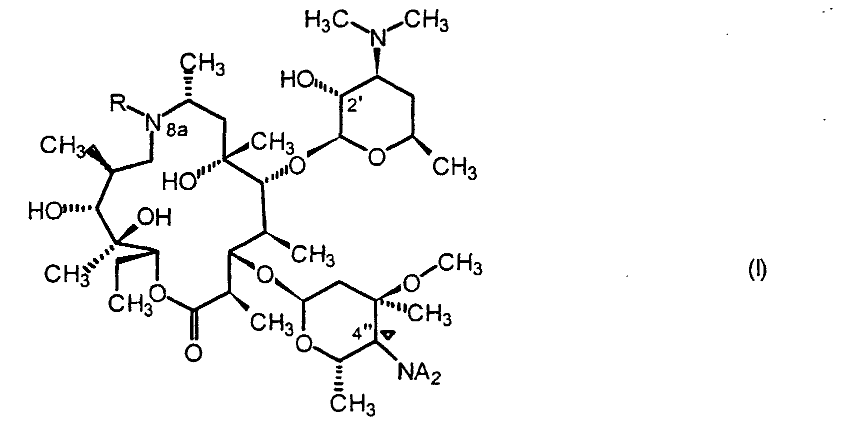 dans laquelle :
- R est un atome d'hydrogène, un groupement alkyle en C₁-C₁₀ , alkényle en C₂-C₁₀ ou arylesulfonyle en C₆-C₁₂, le cas échéant substitués et
- A, identiques ou différents, représentent
   - un atome d'hydrogène,
   - un atome d'azote le cas échéant substitué,
   - un groupement alkyle en C₁-C₄, éventuellement substitué par un ou plusieurs groupements aryles le cas échéant substitués,
   - un groupement R₂CO ou R₂SO₂ avec R₂ représentant un atome d'hydrogène, un groupement alkyle en C₁-C₈ ou aryle, le cas échéant substitués et
- Le symbole ∇ indique qu'il y a eu inversion de configuration au niveau du carbone C- 4" par rapport au composé de formule générale II,
à partir d'un composé de formule générale II 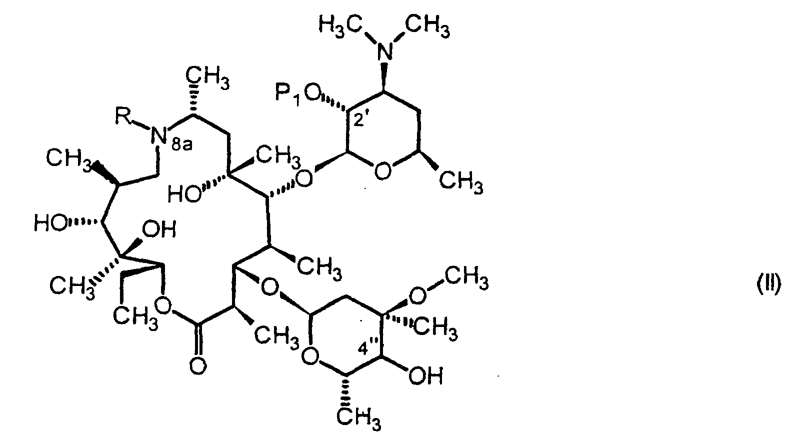 Avec:
- R tel que défini en formule générale I et
- P₁ représentant un groupement protecteur de la fonction hydroxyle en position 2'
caractérisé en ce qu'il comprend au moins les étapes consistant à :
- activer la fonction hydroxyle en position 4" du composé de formule générale II pour obtenir un composé de formule générale III 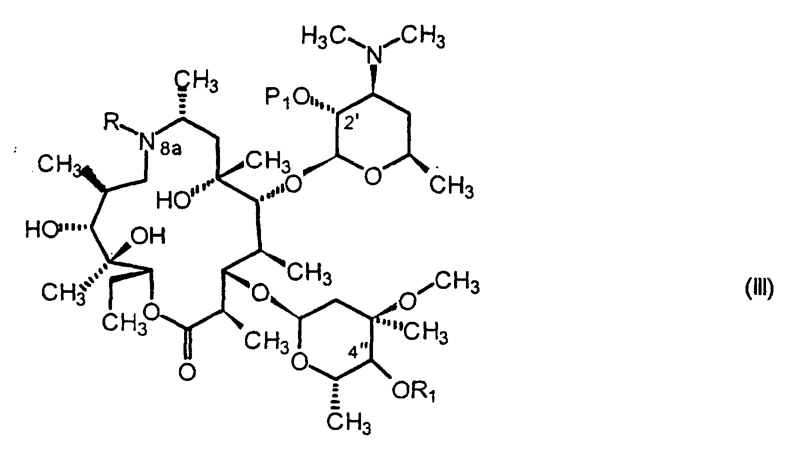 dans laquelle :
   - R et P₁ sont tels que définis en formules générales 1 et II et
   - OR₁ représente un groupe partant,
- à mettre en présence ledit composé de formule générale III ainsi obtenu avec un dérivé nucléophile azoté dans des conditions suffisantes pour permettre le déplacement stéréosélectif de la fonction hydroxyle activée par ledit nucléophile azoté et
- la déprotection de la fonction hydroxyle en position 2',
pour conduire au composé de formule générale I attendu.

Le procédé revendiqué a ainsi pour avantage significatif de ne pas requérir la formation de l'intermédiaire C-4" sp₂ généré obligatoirement dans la voie de synthèse conventionelle discutée précédemment. Il n'implique qu'une inversion de configuration en position 4" et cette inversion est obtenue efficacement par déplacement par un nucléophile azoté, de la fonction alcool activée, présente en cette position 4".

En conséquence, le procédé revendiqué s'avère particulièrement intéressant pour préparer, notamment avec un rendement très satisfaisant, un dérivé 4"-(R)-NA₂ de formule générale I' 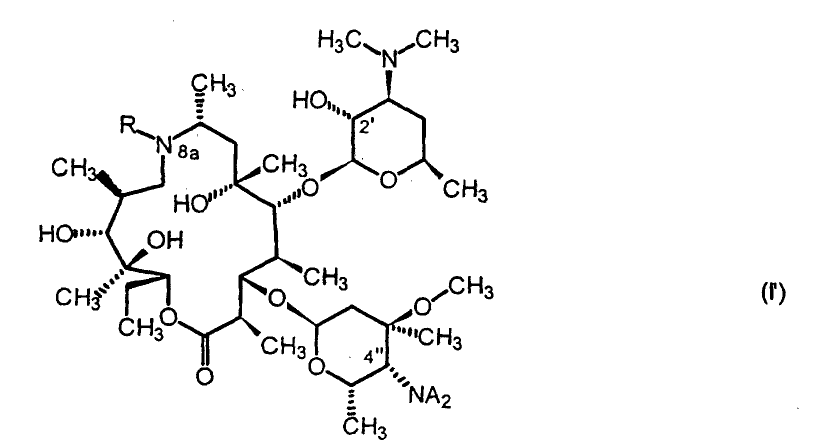 avec A et R tels que définis ci-dessus,
à partir d'un dérivé aza-macrolide 4"(S)-OH de formule générale II' 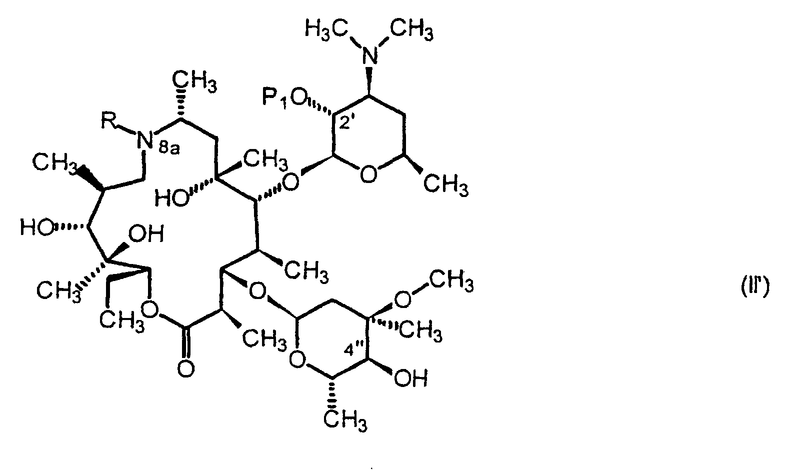 dans laquelle R et P1 sont tels que définis ci-dessus.

En ce qui concerne le groupe partant figuré par OR₁ en formule générale III, il est de préférence choisi parmi les sulfonates d'alkyle en C₁-C₂₀, d'aryle ou d'hétéroaryle en C₅-C₆ ou d'alkylaryle en C₆ à C₂₆, substitués le cas échéant par un ou plusieurs atomes d'halogène, de préférence le fluor et/ou un groupement nitro, cyano ou trifluorométhyle.

De préférence, le groupe partant figuré par OR₁ en formule générale III est un groupement choisi parmi les mésylate, triflate et tosylate et plus préférentiellement est représenté par un groupement triflate.

Comme composé nucléophile azoté, on peut notamment utiliser selon l'invention des composés de type ammoniac, amines substituées ou non par des groupements déprotégeables, comme par exemple un groupement benzyle ou l'un de ses dérivés, amides, imides, sulfonamides, sulfonimides, hydrazines ou azotures.

Selon une variante préférée du procédé revendiqué, il s'agit plus préférentiellement d'un azoture organosoluble non minéral qui peut être généré in situ.

Conviennent tout particulièrement à l'invention, les groupes partants dérivant de l'activation de la fonction hydroxyle en position 4" de la formule générale II par un composé de formule générale IVA ou IVB 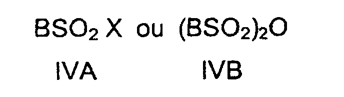 Avec :
- X représentant un atome d'halogène ou un hétérocycle azoté de préférence un cycle imidazole et
- B représentant un groupement sulfonate d'alkyle en C₁-C₂₀, d'aryle ou d'hétéroaryle en C₅-C₆ ou d'alkylaryle en C₆ à C₂₆, substitués ou non par un ou plusieurs atomes d'halogène et de préférence le fluor et/ou un groupement nitro, cyano ou trifluorométhyle.

Selon une variante préférée de l'invention, le composé de formule générale III obtenu par activation avec un composé de formule générale IVA ou IVB est mis en présence d'un azoture organosoluble non minéral pour conduire par déplacement nucléophile stéréosélectif à un composé de formule générale V 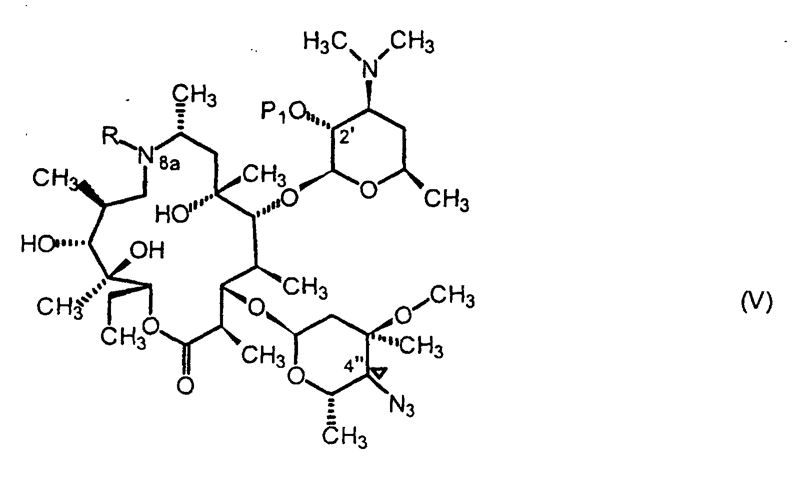 dans laquelle R et P₁ sont tels que définis en formule générale II et le symbole ∇ indique qu'il y a eu inversion de configuration au niveau du carbone C- 4" par rapport au composé de formule générale II

De préférence le carbone C-4" du composé de formule générale II possède une configuration S et celui du composé de formule générale V une configuration R.

Selon cette variante du procédé revendiqué, il peut être en outre réalisé, préalablement ou non à la déprotection de la fonction hydroxyle en position 2', une réduction dudit composé de formule V de manière à obtenir un composé de formule générale I dans laquelle A représente un atome d'hydrogène. Cette réduction de la fonction azoture, peut être réalisée par toute méthode conventionnelle telle que celles décrites par E.F.V. Scriven et al., Chem. Rev. (1988), 88, 297-368. On peut notamment procéder à une réduction catalytique avec de l'hydrogène ou de l'hydrazine en présence de palladium sur charbon par exemple ou de nickel de Raney.

A l'issue de cette réduction, on récupère donc le dérivé amino attendu c'est-à-dire avec inversion de configuration en position 4" et de préférence le dérivé 4"(R)NH₂, avec un rendement satisfaisant.

En conséquence, cette variante du procédé revendiqué est tout particulièrement utile pour préparer des composés de formule générale I" 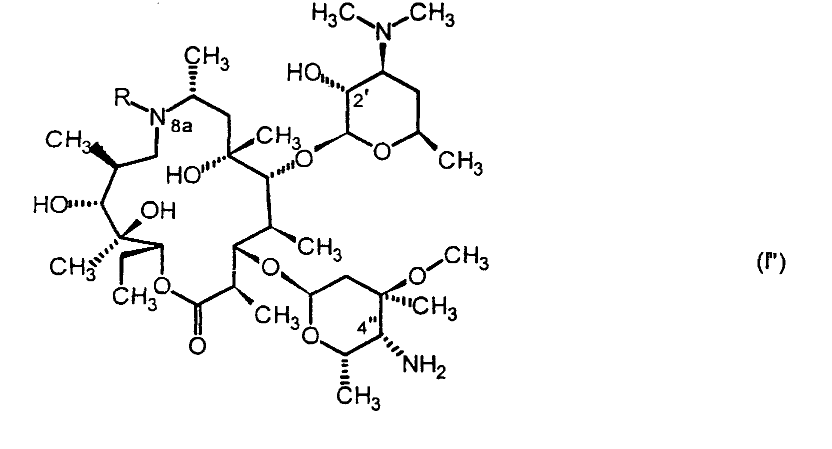 dans laquelle :
- R est un atome d'hydrogène, un groupement alkyle en C₁-C₁₀, alkényle en C₂-C₁₀ ou arylesulfonyle en C₆-C₁₂ le cas échéant substitués, à partir d'un composé de formule générale II' telle que définie ci-dessus.

A titre illustratif des azotures convenant à la présente invention, on peut tout particulièrement citer l'azoture des tétraalkyles en C₁ à C₂₀ ammonium ou phosphonium, des triaryles sulfonium substitués ou non, et des hexaalkyles en C₁ à C₂₀ de guanidinum.

Selon une variante préférée de l'invention, il s'agit d'un azoture tétraalkylammonium et plus particulièrement de tétrabutyl- ou tétraoctylammonium.

Dans un mode de réalisation particulier de l'invention, la formation du dérivé azoture est conduite en milieu biphasique et plus précisément en transfert de phase solide/liquide. Dans ce cas, on génère in situ l'azoture organosoluble à partir d'un azoture minéral tel que l'azoture de sodium et d'un agent de transfert de phase, en présence du composé de formule générale III, dans un solvant organique. L'agent de transfert de phase est de préférence un méthanesulfonate de tétraalkyle en C₁ à C₂₀ ammonium ou phosphonium.

En ce qui concerne le composé de formule générale II, il est généralement obtenu au préalable par protection de la fonction hydroxyle en position 2' du dérivé correspondant. Bien entendu, cette protection est réalisée de manière classique à l'aide d'un groupement protecteur de fonction hydroxyle conventionnel tels ceux figurants dans « Protective groups in organic synthesis » Second Edition Theodora W. Greene, P. G. Wuts, Wiley Intersciences p10-142. Les protocoles de réalisation des opérations, protection et déprotection, sont également décrits dans l'ouvrage référencé ci-dessus.

Consécutivement à cette protection de la fonction hydroxyle en position 2' on réalise l'activation de la fonction hydroxyle en position 4". Cette activation du composé de formule générale II est également réalisée dans des conditions opératoires usuelles telles que celles décrites dans « Protective groups in organic synthesis » Second Edition Theodora W. Greene, P. G. M. Wuts, Wiley Intersciences p117-118. Les exemples soumis ci-après rendent compte d'un protocole détaillé pour l'activation de la fonction hydroxyle 4" par l'anhydride triflique.

En ce qui concerne le déplacement nucléophile du groupement partant en position 4", il est effectué dans un solvant organique de préférence anhydre. Dans la variante préférée de l'invention mettant en oeuvre un azoture organosoluble, conviennent notamment à titre de solvants les solvants aromatiques comme le benzène et le toluène ou les éthers comme le THF ou le méthylterbutyléther.

Le composé nucléophile azoté et de préférence l'azoture est utilisé à raison d'environ 1 à 30 équivalents par rapport au composé de formule III et de préférence à raison d'environ 1 à 5 équivalents.

Classiquement, la température est comprise entre - 20 et 180°C. En règle générale, elle est ajustée de manière à privilégier la cinétique de la réaction sans porter préjudice à la stabilité des composés.

Selon une variante préférée de l'invention, on réalise dans la première étape l'activation de la fonction hydroxyle en position 4" par un groupement trifluorométhanesulfonate et l'on procède à la substitution nucléophile avec inversion de configuration avec l'azoture de tétrabutyl- ou tétraoctyl-ammonium dans le toluène et à température ambiante.

Selon une variante préférée de l'invention, R représente un groupement méthyle dans les formules générales I, I', I", II, II', III et V et A un atome d'hydrogène dans la formule générale I et I'.

La présente invention a également pour objet les composés de formule générale VI 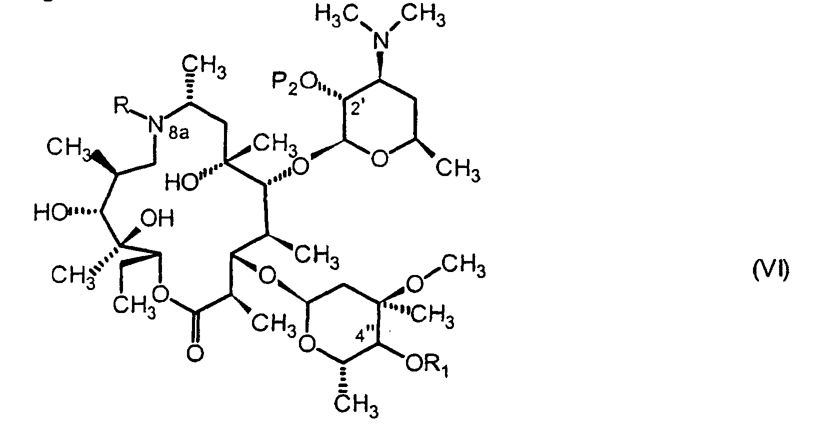 dans laquelle
- P₂ représente un atome d'hydrogène ou un groupement protecteur,
- R représente un atome d'hydrogène, un groupement alkyle en C₁-C₁₀ , alkényle en C₂-C₁₀ ou arylesufonyle en C₆-C₁₂ le cas échéant substitués et
- OR₁ représente un groupe partant à titre d'intermédiaire pour la préparation d'un composé de formule générale 1.

De préférence, R représente un groupement méthyle, OR₁ représente un groupement triflate et plus préférentiellement le carbone C-4" possède une configuration S.

La présente invention se rapporte également aux composés de formule générale VII 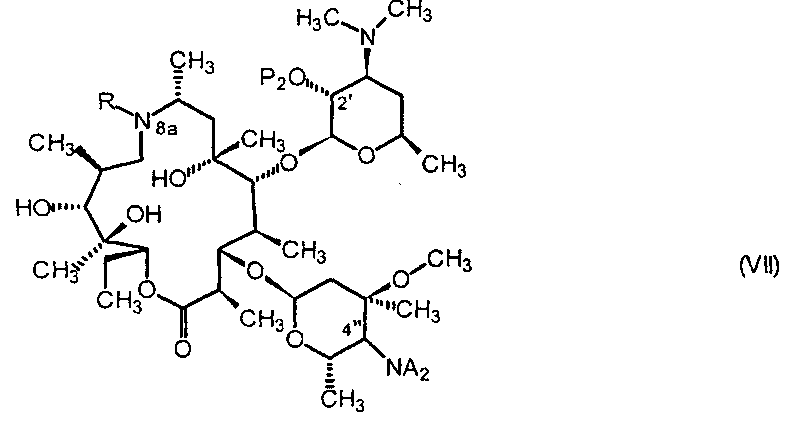 dans laquelle
- P₂ représente un atome d'hydrogène ou un groupement protecteur
- R représente un atome d'hydrogène, un groupement alkyle en C₁-C₁₀ , alkényle en C₂-C₁₀ ou arylesulfonyle en C₆-C₁₂ le cas échéant substitués et
- A, identiques ou différents, représentent
   - un atome d'azote le cas échéant substitué,
   - un groupement alkyle en C₁-C₄, éventuellement substitué par un ou plusieurs groupements aryles le cas échéant substitués,
à titre d'intermédiaire pour la préparation d'un composé de formule générale I.

De préférence, R représente un groupement méthyle et NA₂ un groupement N₃ et plus préférentiellement le carbone C-4" possède une configuration R.

Les exemples figurant ci-après sont présentés à titre illustratif et non limitatif de la présente invention.

### EXEMPLE 1

### Préparation du composé 4"-deoxy-4"(R)-amino-2'-acetoxy-9-deoxo-8a-aza-8a-méthyl-8a-homoerythromycine A:

Le schéma de synthèse retenu est le suivant :

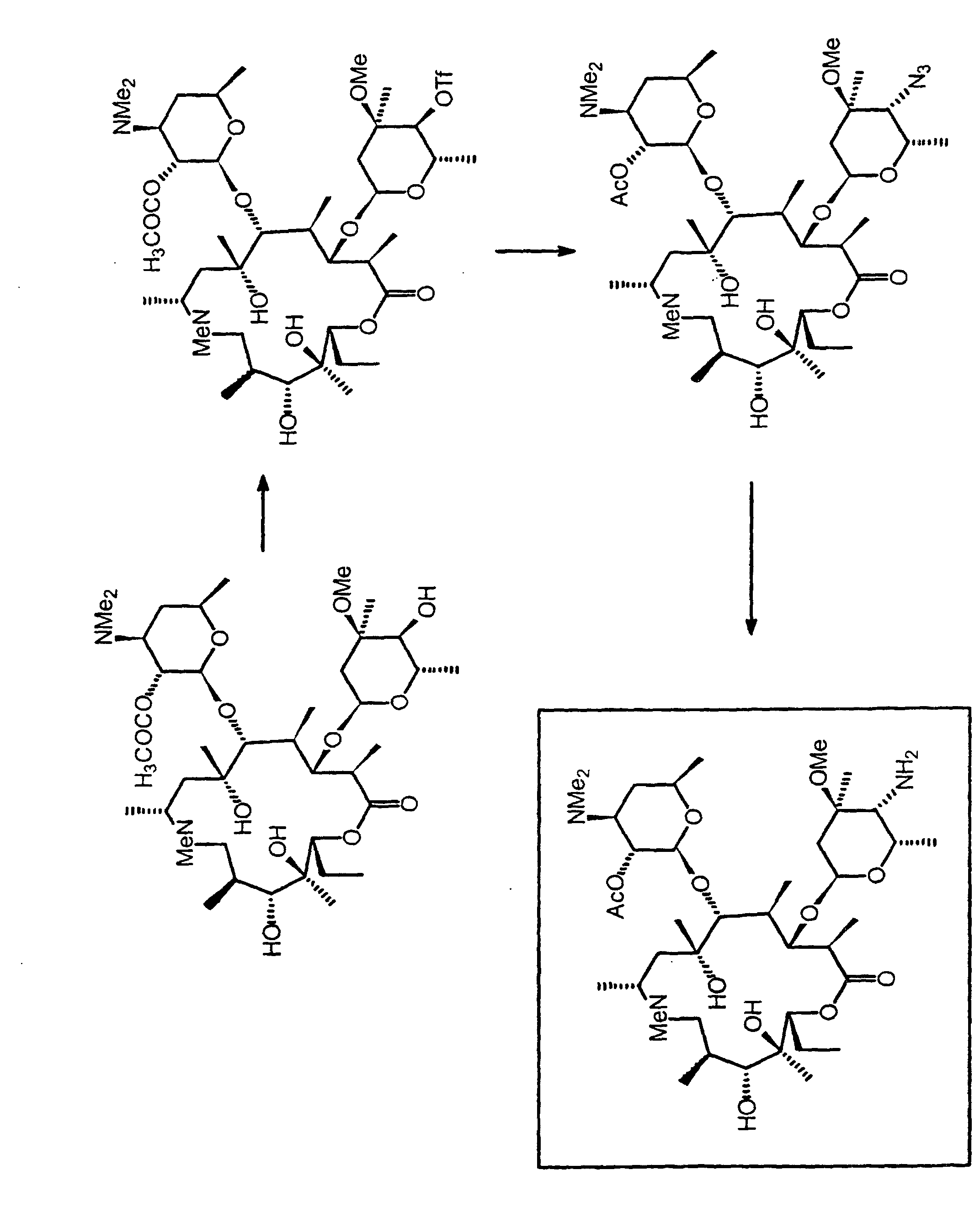

Tous les essais sont réalisés sous atmosphère inerte.

### 1) formation du 4"(S)-trifluorométhanesulfonate-2'-acetoxy-9-deoxo-8a-aza-8a-méthyl-8a-homoerythromycine A:

A une solution d'alcool 2'-acetoxy-9-deoxo-8a-aza-8a-méthyl-8a-homoerythromycine A (0.1 g, 0,12 mmol, 1 équiv.) dans du dichlorométhane anhydre (0,4 ml) est ajoutée de la pyridine (39,5 mg, 0,51 mmol, 5 équiv.). La solution est refroidie à 0°C puis une solution d'anhydride triflique (42,3 mg, 0,15 mmol, 1,2 équiv.) est ajoutée au goutte à goutte. La solution est agitée 1 h à 0°C puis 30 min à température ambiante. Après dilution du mélange réactionnel avec du dichlorométhane anhydre (10 ml), le mélange réactionnel est refroidi à 0°C puis hydrolysé par addition d'une solution aqueuse saturée de bicarbonate de sodium (10 ml). La phase organique est séparée puis lavée avec de l'eau distillée (10 ml), séchée sur sulfate de magnésium et évaporée. Le brut est repris par de l'heptane (10 ml) pour éliminer toute trace de pyridine résiduelle par distillation azéotropique. On obtient 110,4 mg de 4"(S)-trifluorométhanesulfonate-2'-acetoxy-9-deoxo-8a-aza-8a-méthyl-8a-homoerythromycine A de pureté supérieure ou égale à 90%. La structure est confirmée par analyse RMN et SM.

### 2) formation du 4"-deoxy-4"(R)-azido-2'-acetoxy-9-deoxo-8a-aza-8a-méthyl-8a-homoerythromycine A:

Au 4"(S)-trifluorométhanesulfonate-2'-acetoxy-9-deoxo-8a-aza-8a-méthyl-8a-homoerythromycine A non purifié de l'étape précédente (1,84 g, 2,0 mmol, 1 équiv.) à température ambiante est ajoutée une solution 0,58 M d'azoture de tétrabutylammonium dans du toluène (4,5 ml; env. 1,3 équiv.). Le mélange réactionnel est agité 3 jours à température ambiante puis dilué avec du toluène (25 ml). Cette solution est lavée trois fois avec de l'eau distillée (3x10 ml) puis séchée sur sulfate de magnésium et évaporée. On obtient 1,63 g de 4"-deoxy-4"(R)-azido-2'-acetoxy-9-deoxo-8a-aza-8a-méthyl-8a-homoerythromycine A de pureté 70%. La structure est confirmée par analyse RMN et SM.

### 3) formation du composé 4"-deoxy-4"(R)-amino-2-acetoxy-9-deoxo-8a-aza-8a-méthyl-8a-homoerythromycine A:

A une solution de 4"-deoxy-4"(R)-azido-2'-acetoxy-9-deoxo-8a-aza-8a-méthyl-8a-homoerythromycine A non purifié de l'étape précédente (250,0 mg, 0,30 mmol, 1 équiv.) dans de l'isopropanol (5 ml) est ajoutée du nickel de Raney (200 mg). De l'hydrazine monohydrate (30 microlitre, 0,6 mmol, 2 équiv.) est additionnée toutes les 30 minutes. Le temps de réaction est de 2h. Le mélange réactionnel est dilué avec de l'acétate d'éthyle (10 ml) et filtré. Le filtrat est lavé avec une solution aqueuse saturée de bicarbonate de sodium (10 ml) puis avec de l'eau (10 ml). Après séchage sur sulfate de magnésium, le filtrat est évaporé. On obtient 230 mg de 4"-deoxy-4"(R)-amino-2'-acetoxy-9-deoxo-8a-aza-8a-méthyl-8a-homoerythromycine A de pureté 60%. La structure est confirmée par analyse RMN et SM.

### EXEMPLE 2

A une solution de 4"(S)-trifluorométhanesulfonate-2'-acetoxy-9-deoxo-8a-aza-8a-méthyl-8a-homoerythromycine A (92,3 mg, 0,1 mmol, 1 équiv.) dans du toluène (0,2 ml) est ajoutée à température ambiante l'azoture de tetraoctylammonium (190,3 ml, 0,5mmol, 5 équiv.). Après deux jours d'agitation à température ambiante, on rajoute de l'azoture de tetraoctylammonium (58 mg, 0,15 mmol, 1,5 équiv.). Après deux jours supplémentaires d'agitation à température ambiante, le mélange réactionnel est dilué avec du toluène (10 ml) et lavé avec de l'eau (10 ml). La phase organique est séparée et séchée sur sulfate de sodium. Après évaporation des solvants, l'analyse RMN-¹H indique la présence majoritaire du composé 4"-deoxy-4"(R)-azido-2'-acetoxy-9-deoxo-8a-aza-8a-méthyl-8a-homoerythromycine A.

### EXEMPLE 3

A une solution de 4"(S)-trifluorométhanesulfonate-2'-acetoxy-9-deoxo-8a-aza-8a-méthyl-8a-homoerythromycine A (185 mg, 0,2 mmol, 1 équiv.) dans du toluène (0,4 ml) à température ambiante sont successivement ajoutés le méthanesulfonate de tetrabutylphosphonium (355 mg, 1 mmol, 5 équiv.) puis l'azoture de sodium (325 mg, 5 mmol, 25 équiv.). Après trois jours d'agitation à température ambiante, le mélange réactionnel est dilué avec du toluène (10 ml) et lavé à l'eau (10 ml). La phase organique est séparée et séchée sur sulfate de sodium. Après évaporation des solvants, l'analyse RMN-¹H indique la présence majoritaire du composé 4"-deoxy-4"(R)-azido-2'-acetoxy-9-deoxo-8a-aza-8a-méthyl-8a-homoerythromycine A.

### EXEMPLE 4

A une solution de 4"(S)-trifluorométhanesulfonate-2'-acetoxy-9-deoxo-8a-aza-8a-méthyl-8a-homoerythromycine A (92 mg, 0,1 mmol, 1 équiv.) dans du toluène (0,25 ml) à température ambiante sont successivement ajoutés le méthanesulfonate de tetraoctylammonium (217 mg, 0,38 mmol, 3,8 équiv.) puis l'azoture de tetrabutylammonium (158 mg, 2,5 mmol, 25 équiv.). Après 4 jours de réaction à température ambiante, le mélange réactionnel est dilué avec du toluène (10 ml) et lavé à l'eau (10 ml). La phase organique est séparée et séchée sur sulfate de sodium. Après évaporation des solvants, l'analyse RMN-¹H indique la présence majoritaire du composé 4"-deoxy-4"(R)-azido-2'-acetoxy-9-deoxo-8a-aza-8a-méthyl-8a-homoerythromycine A.

### EXEMPLE 5

Une solution de la 4"(S)-trifluorométhanesulfonate-2'-acetoxy-9-deoxo-8a-aza-8a-méthyt-8a-homoerythromycine A (21,4 mg, 0.023 mmol) dans la N-méthylpyrrolidinone est saturée avec de l'ammoniac gazeux. Cette solution est agitée 48h à température ambiante. Le mélange réactionnel est ensuite dilué avec de l'acétate d'éthyle (10 ml) et lavé à l'eau (15 ml). La phase organique est séparée, séchée sur sulfate de sodium et évaporé. L'analyse LC/MS indique la formation de 22% en normalisation interne de 4"-deoxy-4"(R)-amino-2'-acetoxy-9-deoxo-8a-aza-8a-méthyl-8a-homoerythromycine A.

## Revendications

1. Procédé de préparation stéréosélectif d'un composé de formule générale I 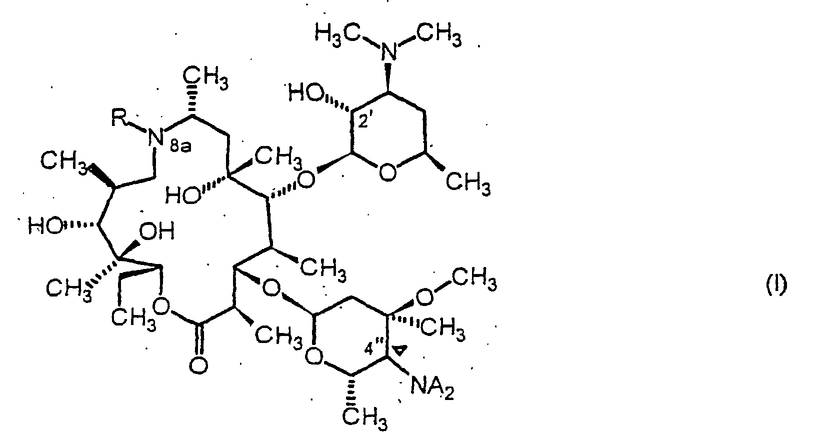 dans laquelle :
- R est un atome d'hydrogène, un groupement alkyle en C₁-C₁₀, alkényle en C₂-C₁₀ ou arylesulfonyle en C₆-C₁₂ le cas échéant substitués et
- A, identiques ou différents, représentent
• un atome d'hydrogène,
• un atome d'azote le cas échéant substitué,
• un groupement alkyle en C₁-C₄, éventuellement substitué par un ou plusieurs groupements aryles le cas échéant substitués,
• un groupement R₂CO ou R₂SO₂ avec R₂ représentant un atome d'hydrogène, un groupement alkyle en C₁-C₈ ou aryle le cas échéant substitués et
- Le symbole ∇ indique qu'il y a eu inversion de configuration au niveau du carbone C- 4" par rapport au composé de formule générale II,
à partir d'un composé de formule générale II 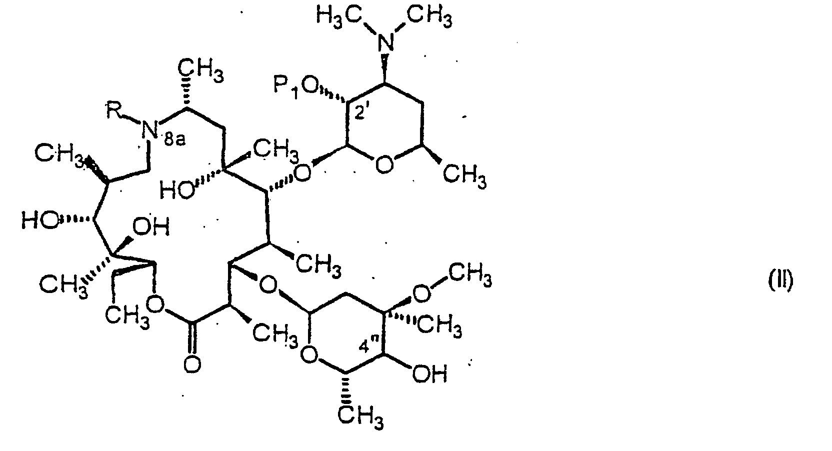
- avec R tel que défini en formule générale 1 et
- P₁ représentant un groupement protecteur de la fonction hydroxyle en position 2'
**caractérisé en ce qu'**il comprend au moins les étapes consistant à :
- activer la fonction hydroxyle en position 4" du composé de formule générale II pour obtenir un composé de formule générale III 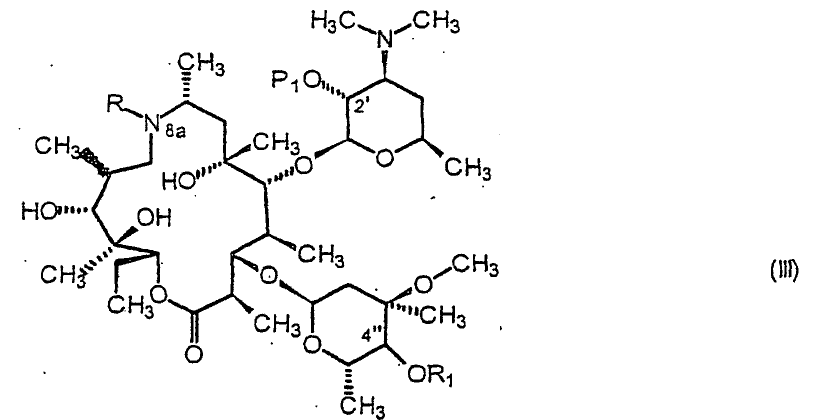
dans laquelle:
- R et P₁ sont tels que définis en formules générales et II et
- OR₁ représente un groupe partant,
- à mettre en présence ledit composé de formule générale III ainsi obtenu avec un dérivé nucléophile azoté dans des conditions suffisantes pour permettre le déplacement stéréosélectif de la fonction hydroxyle activée par ledit nucléophile azoté et
- la déprotéction de la fonction hydroxyle en position 2', pour conduire au composé de formule générale I attendu.

2. Procédé selon la revendication 1, **caractérisé en ce que** l'on prépare un dérivé 4"-(R)-NA₂ de formule générale I'. 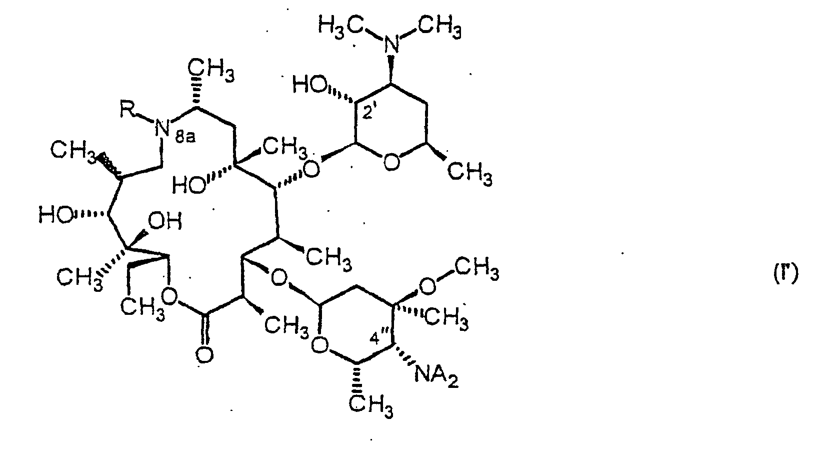 avec A et R étant tels que définis en revendication 1
à partir d'un dérivé aza-macrolide 4"(S)-OH de formule générale II' 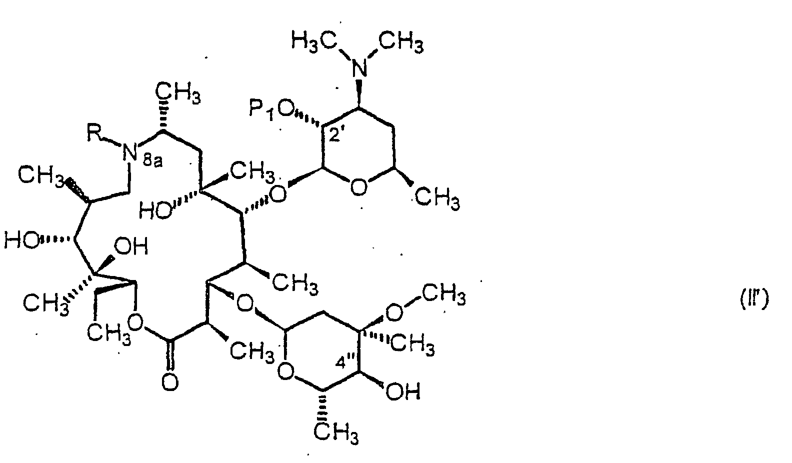 dans laquelle R et P1 sont tels que définis en revendication 1.

3. Procédé selon la revendication 1 ou 2 **caractérisé en ce que** le groupe partant figuré par OR₁ en formule générale III est choisi parmi les sulfonates d'alkyle en C₁-C₂₀,-d'aryle ou d'hétéroaryle en C₅-C₆ ou d'alkylaryle en C₆-C₂₆, substitués le cas échéant par un ou plusieurs atomes d'halogène, de préférence le fluor et/ou un groupement nitro, cyano ou trifluorométhyle.

4. Procédé selon l'une des revendications 1 à 3 **caractérisé en ce que** le groupe partant figuré par OR₁ en formule générale III est un groupement triflate.

5. Procédé selon l'une des revendications 1 à 4 **caractérisé en ce que** le groupe partant dérivé de l'activation de la fonction hydroxyle en position 4" de la formule générale Il par un composé de formule IVA ou IVB 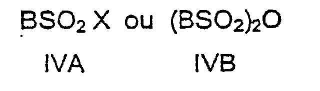 Avec :
- X représentant un atome d'halogène ou un hétérocycle azoté de préférence un cycle imidazole et
- B représentant un groupement sulfonate d'alkyle en C₁-C₂₀, d'aryle ou d'hétéroaryle en C₅-C₆ ou d'alkylaryle en C₆-C₂₆, substitués le cas échéant par un ou plusieurs atomes d'halogène, de préférence le fluor et/ou un groupement nitro, cyano ou trifluorométhyle.

6. Procédé selon l'une des revendications 1 à 5, **caractérisé en ce que** le composé nucléophile azoté est choisi parmi l'ammoniac, les amines substituées ou non par des groupements déprotégeables, amides, imides, sulfonamides, sulfonimides, hydrazines ou azotures.

7. Procédé selon l'une des revendications précédentes, **caractérisé en ce que** le composé nucléophile azoté est utilisé à raison d'environ 1 à 30 équivalents par rapport au composé de formule générale III.

8. Procédé selon l'une des revendications 1 à 7 **caractérisé en ce que** le composé nucléophile azoté est un azoture organosoluble non minera), généré le cas échéant in situ.

9. Procédé selon la revendication 8, **caractérisé en ce que**
- le composé de formule générale II est activé avec un composé de formule générale IVA ou IVB telles que définies en revendication 5,
- Le composé de formule générale III ainsi obtenu est ensuite mis en présence d'un azoture organosoluble non minéral pour conduire par déplacement nucléophile stéréosélectif à un composé de formule générale V 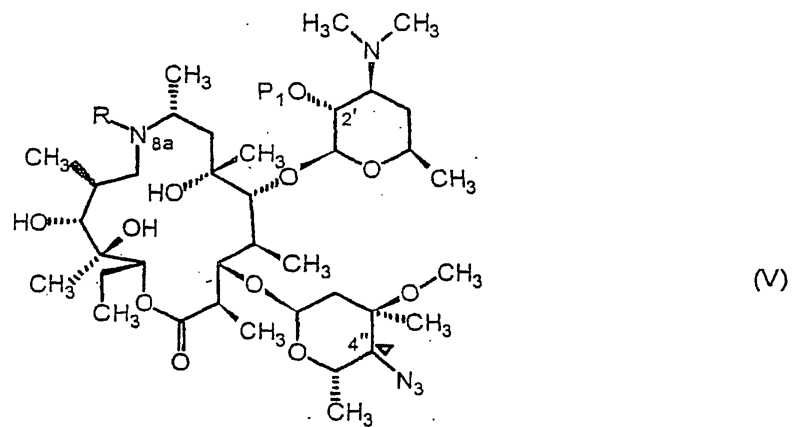
dans laquelle R et P₁ sont tels que définis en formule générale II et le symbole ∇ indique qu'il y a eu inversion de configuration au niveau du carbone C- 4" par rapport au composé de formule générale II.

10. Procédé selon la revendication 9, **caractérisé en ce qu** 'il est réalisé en outre une réduction dudit composé de formule V de manière à obtenir un composé de formule générale I dans laquelle A représente un atome d'hydrogène.

11. Procédé selon la revendication 9 ou 10 **caractérisé en ce que** le carbone C-4" du composé de formule générale Il possède une configuration S et celui du composé de formule générale V une configuration R.

12. Procédé selon l'une des revendications 8 à 11, **caractérisé en ce que** l'azoture organosoluble non mineral est choisi parmi l'azoture des tétraalkyles en C₁ à C₂₀ ammonium ou phosphonium, des triaryles suffohium substitués ou non, et des hexaalkyles en C₁ à C₂₀ de guanidinum.

13. Procédé selon l'une des revendications 8 à 12, **caractérisé en ce que** l'azoture est l'azoture de tétrabutylammonium ou l'azoture de tétraoctylammonium.

14. Procédé selon l'une des revendications 8 à 13, **caractérisé en ce que** le déplacement nucléophile du groupe partant en position 4" par un azoture organosoluble non minéral est réalisé au sein d'un solvant choisi parmi les solvants aromatiques comme le benzène et le toluène et les éthers comme le méthylterbutyléther et le THF.

15. Procédé selon l'une des revendications précédentes, **caractérisé en ce que** l'on réalise dans la première étape l'activation de la fonction hydroxyle en position 4" par un groupement triflurométhanesutfonate et l'on procède à la substitution nucléophile avec inversion de configuration avec l'azoture de tétrabutyl- ou tétraoctyl-ammonium dans le toluène, à température ambiante.

16. Procédé selon l'une des revendications précédentes, **caractérisé en ce que** R représente un groupement méthyle dans les formules générales I, I', I", II, II', III et V et A un atome d'hydrogène dans les formules générales I et I'.

17. Composé de formule générale VI 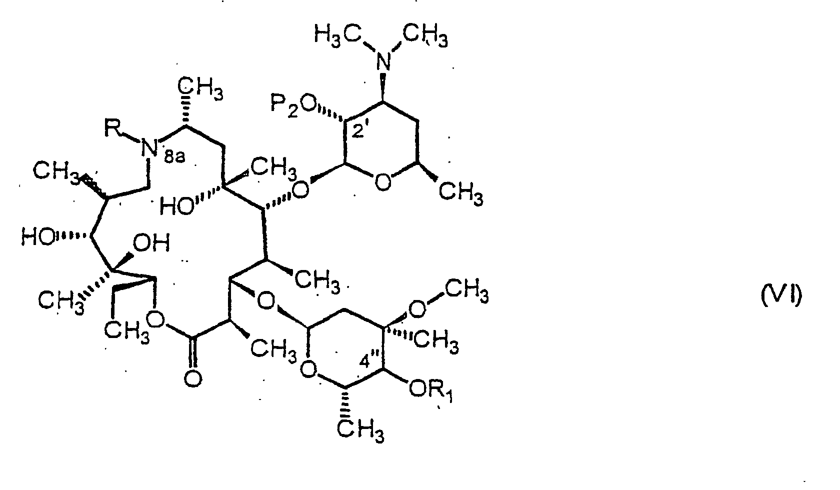 dans laquelle
- P₂ représente un atome d'hydrogène ou un groupement protecteur,
- R représente un atome d'hydrogène, un groupement alkyle en C₁-C₁₀, alkényle en C₂-C₁₀ ou arylesulfonyle en C₆-C₁₂ le cas échéant substitués et
- OR₁ représente un groupe partant
à titre d'intermédiaire pour la préparation d'un composé de formule générale I.

18. Composé de formule générale VI selon la revendication 17, **caractérisé en ce que** R représente un groupement méthyle et OR₁ un groupement triflate.

19. Composé de formule générale VI selon la revendication 17 ou 18 **caractérisé en ce que** le carbone C-4" possède une configuration S.

20. Composé de formule générale VII 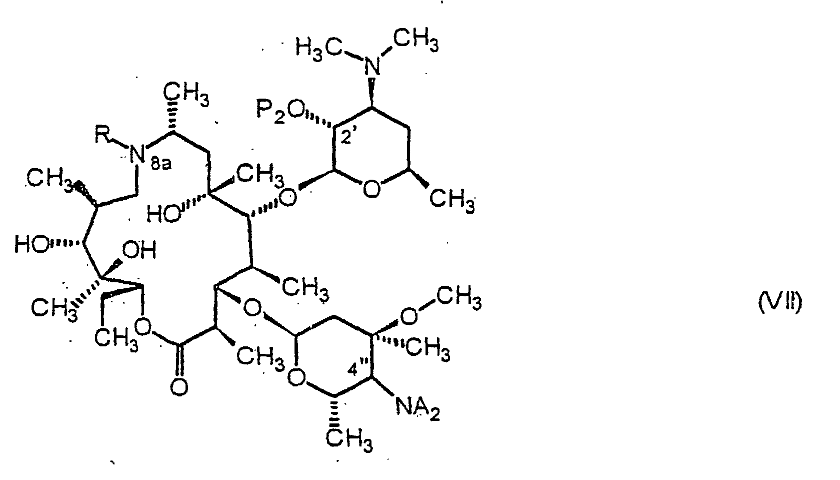 dans laquelle .
- P₂ représente un atome d'hydrogène ou un groupement protecteur
- R est un atome d'hydrogène, un groupement alkyle en C₁-C₁₀, alkényle en C₂-C₁₀ ou arylesulfonyle en C₆-C₁₂ le cas échéant substitués et
- A, identiques ou différents, représentent
• un atome d'azote le cas échéant substitué,
• un groupement alkyle en C₁-C₄ substitué par un ou plusieurs groupements aryles le cas échéant substitués,
à titre d'intermédiaire pour la préparation d'un composé de formule générale I.

21. Composé de formule générale VII selon la revendication 20, **caractérisé en ce que** R représente un groupement méthyle et NA₂ un groupement N₃.

22. Composé de formule générale VII selon la revendication 20 ou 21 **caractérisé en ce que** le carbone C-4" possède une configuration R.

## Patentansprüche

1. Verfahren zur stereoselektiven Herstellung einer Verbindung der allgemeinen Formel I 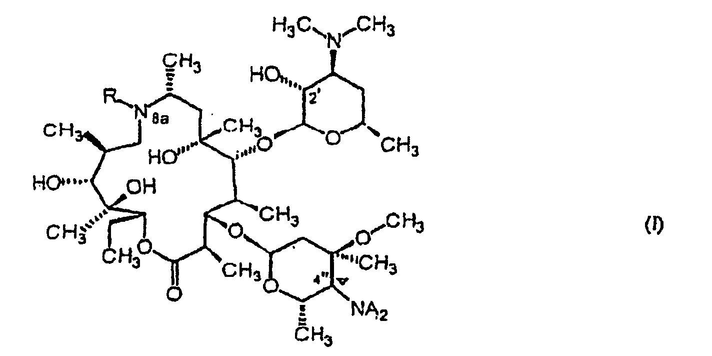 n welcher:
- R ein Wasserstoffatom, eine Alkylgruppe mit C₁- C₁₀, eine Alkenylgruppe mit C₂- C₁₀ oder eine Arylsulfonylgruppe mit C₆ - C₁₂, gegebenenfalls substituiert ist und
- A identisch oder verschieden aufweisen
• ein Wasserstoffatom,
• ein Stickstoffatom, gegebenenfalls substituiert,
• eine Alkylgruppe mit C₁ .- C₄, eventuell substituiert durch eine oder mehrere Alkylgruppen, die gegebenenfalls substituiert sind,
• eine R₂CO- oder R₂SO₂-Gruppe, wobei R₂ ein Wasserstoffatom einer Alkylgruppe mit C₁- C₆ oder Arylgruppe aufweist, gegebenenfalls substituiert und
- das Symbol ∇ anzeigt, daß eine inversion der Konfiguration an dem Kohlenstoff C - 4" bezüglich der Verbindung der allgemeinen Formel 11 stattgefunden hat,
ausgehend von einer Verbindung der allgemeinen Formel II 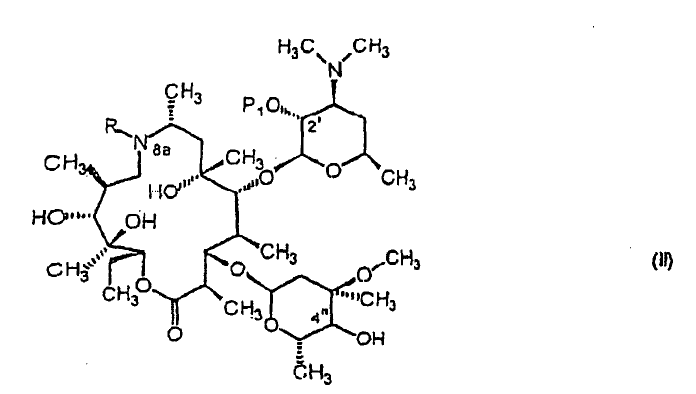
- mit R wie in der allgemeinen Formel I definiert ist und
- wobei P₁ eine Schutzgruppe der Hydroxylfunktion in Position 2' darstellt,
**dadurch gekennzeichnet, daß** es mindestens die Schritte aufweist, welche darin bestehen:
- die Hydroxylfunktion in Position 4" der Verbindung der allgemeinen Formel II zu aktivieren, um eine Verbindung der allgemeinen Formel III zu erhalten: 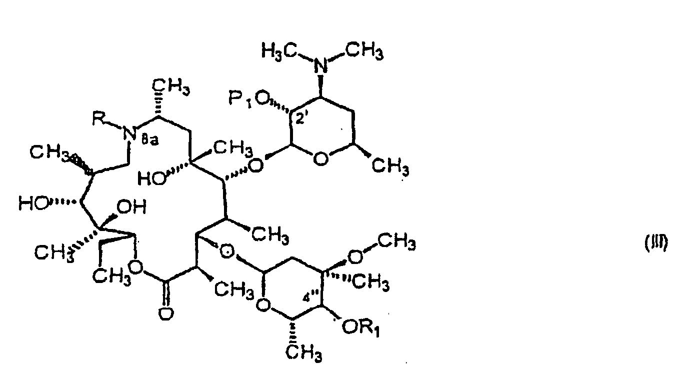 in welcher
- R und P₁ so sind, wie in den allgemeinen Formeln I und II definiert ist, und
- OR₁ eine austretende Gruppe aufweist,
- die Verbindung der so erhaltenen allgemeinen Formel III mit einem nukleophilen Stickstoffderivat unter Bedingungen in Anwesenheit zu bringen, die ausreichen, die stereoselektive Umlagerung der Hydroxylfunktion zu erlauben, welche durch den nukleophilen Stickstoff aktiviert wurde, und
- die Entfernung des Schutzes der Hydroxylfunktion in Position 2', um zu der Verbindung der erwarteten allgemeinen Formel I hinzuführen.

2. Verfahren nach Anspruch 1, **dadurch gekennzeichnet, daß** man ein Derivat 4"-®-NA₂ der allgemeinen Formel I' herstellt: 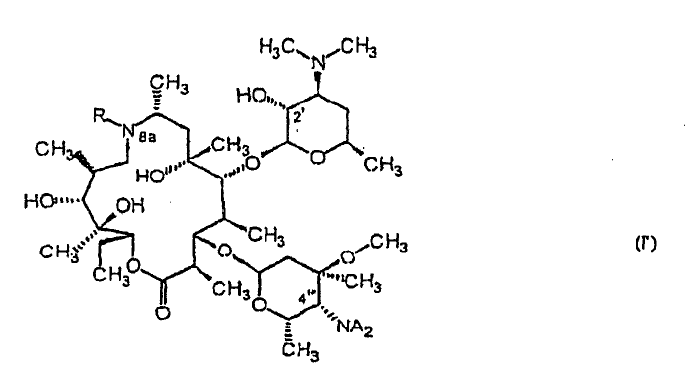 wobei A und R so sind, wie in Anspruch 1 definiert ist, ausgehend von einem Aza-Makrolid 4"(S)-OH der allgemeinen Formel II' 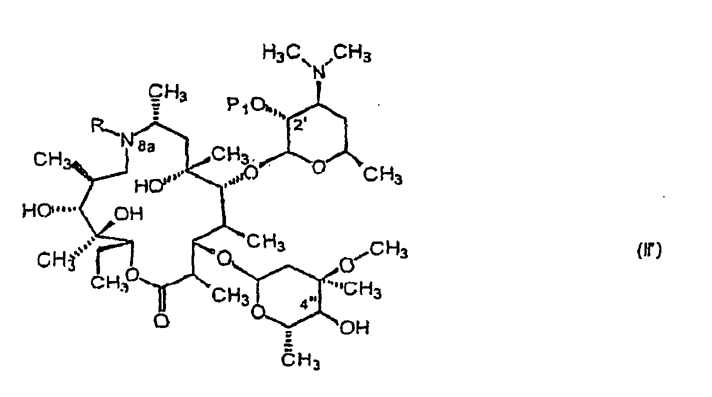 in weicher R und P 1 so sind, wie in Anspruch 1 definiert ist.

3. Verfahren nach Anspruch 1 oder 2, **dadurch gekennzeichnet, daß** die austretende Gruppe, welche durch OR₁ mit der allgemeinen Formel III dargestellt ist, unter den Alkylsulfonaten mit C₁ - C₂₀. Aryl- oder Ffeteroarylsutfonaten mit C₅ - C₆ oder Alkylarylsulfonaten mit C₆ - C₂₆ gewählt sind, gegebenenfalls durch eines oder mehrere Halogenatome ersetzt, vorzugsweise Fluor und/oder eine Nitrogruppe, Cyanogruppe oder Trifluormethylgruppe.

4. Verfahren nach einem der Ansprüche 1 bis 3, **dadurch gekennzeichnet. daß** die austretende Gruppe, welche durch OR₁ mit der allgemeinen Formel III dargestellt ist, eine Triflatgruppe ist.

5. Verfahren nach einem der Ansprüche 1 bis 4, **dadurch gekennzeichnet, daß** die austretende Gruppe von der Aktivierung der Hydroxylfunktion in Position 4" der allgemeinen Formel II durch eine Verbindung der Formel IVA oder IVB abstammt 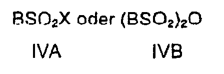 wobei
- X ein Halogenatom oder eine heterozyklische Stickstoffverbindung, vorzugsweise einen Imidazolring aufweist, und
- B eine Alkylsulfonatgruppe mit C₁- C₂₀, Arylgruppe oder Heteroaryigruppe mit C₅ - C₆ oder Alkylarylgruppe mit C₆ - C₂₆ aufweist, gegebenenfalls durch ein oder mehrere Halogenatome substituiert, vorzugsweise Fluor und/oder eine Nitrogruppe, Cyanogruppe oder Trifluormethylgruppe.

6. Verfahren nach einem der Ansprüche 1 bis 5, **dadurch gekennzeichnet, daß** die nukleophile Stickstoffverbindung unter dem Ammoniak, den substituierten Aminen oder nicht den Schutz entfernenden Gruppen, den Amiden, den Imiden, den Sulfonamiden, den Sulfonimiden, den Hydrazinen oder den Aziden bzw. den Nitriden ausgesucht ist.

7. Verfahren nach einem der vorhergehenden Ansprüche, **dadurch gekennzeichnet, daß** die nukleophile Stickstoffverbindung in der Größe von etwa 1 bis 30 Äquivalenten bezüglich der Verbindung der allgemeinen Formel III verwendet wird.

8. Verfahren nach einem der Ansprüche 1 bis 7,**dadurch gekennzeichnet, daß** die nukleophile Stickstoffverbindung ein organlösliches, nicht-mineralisches Azid ist, das gegebenenfalls in situ erzeugt wird.

9. Verfahren nach Anspruch 8, **dadurch gekennzeichnet, daß**
- die Verbindung der allgemeinen Formel II mit einer Verbindung der allgemeinen Formel IVA oder IVB, wie zum Beispiel in Anspruch 5 definiert ist, aktiviert wird,
- die so erhaltene Verbindung der allgemeinen Formel III anschließend in Gegenwart von organlöslichem, nicht-mineralischem Azid gebracht wird, um durch stereoselektive, nukleophile Umlagerung zu einer Verbindung der allgemeinen Formel V hinzuführen 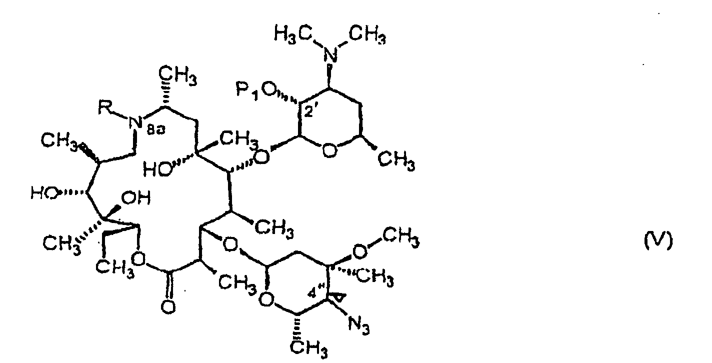
- in welcher R und P₁ so sind, wie in der allgemeinen Formel II definiert ist, und das Symbol ∇ anzeigt, daß eine Inversion der Konfiguration bei dem Kohlenstoff C-4" bezüglich der Verbindung der allgemeinen Formel II stattgefunden hat.

10. Verfahren nach Anspruch 9, **dadurch gekennzeichnet, daß** ferner eine Reduktion der Verbindung der Formel V derart durchgeführt wurde, daß eine Verbindung der allgemeinen.Formet I erhalten wird, in welcher A ein Wasserstoffatom darstellt.

11. Verfahren nach Anspruch 9 oder 10, **dadurch gekennzeichnet, daß** der Kohlenstoff C-4" der Verbindung der allgemeinen Formel II eine Konfiguration S besitzt und derjenige der Verbindung der allgemeinen Formel V eine Konfiguration R besitzt.

12. Verfahren nach einem der Ansprüche 8 bis 11, **dadurch gekennzeichnet, daß** das organisch lösliche, nicht-mineralische Azid unter den Aziden der Ammonium- oder Phosphoniumtetraal-kylene mit C₁ bis C₂₀, den substituierten oder nicht-substitulerten Sultonlumtrlarylen und den Hexaalkylen mit C₁ bis C₂₀ des Guanidinium ausgewählt ist.

13. Verfahren nach einem der Ansprüche 8 bis 12, **dadurch gekennzeichnet, daß** das Azid Tetrabutylammoniumazid oder Tetraoctylammoniumazid ist.

14. Verfahren nach einem der Ansprüche 8 bis 13, **dadurch gekennzeichnet, daß** die nukleophile Umlagerung der austretenden Gruppe In Position 4" durch ein organisch lösliches nicht-mineralisches Azid in einem Lösungsmittel realisiert wird, das unter den aromatischen Lösungsmitteln, wie dem Benzol, dem Toluol und den Ethern, wie Methyltertiärbutylether und dem THF gewählt ist

15. Verfahren nach einem der vorhergehenden Ansprüche, **dadurch gekennzeichnet, daß** man in dem ersten Schritt die Aktivierung der Hydroxylfunktion in Position 4" durch eine Trifluormethansuifonatgruppe realisiert und man die nukleophile Substitution mit Inversion der Konfiguration mit dem Azid von Tetrabutyl- oder Tetraoctyl-Ammonium in dem Toluol bei Umgebungstemperatur durchführt.

16. Verfahren nach einem der vorhergehenden Ansprüche, **dadurch gekennzeichnet, daß** R eine Methylgruppe in den allgemeinen Formeln I, I', I", II, II', III und V und A ein Wasserstoffatom in den allgemeinen Formeln I und I' aufweist.

17. Verbindung der allgemeinen Formel VI 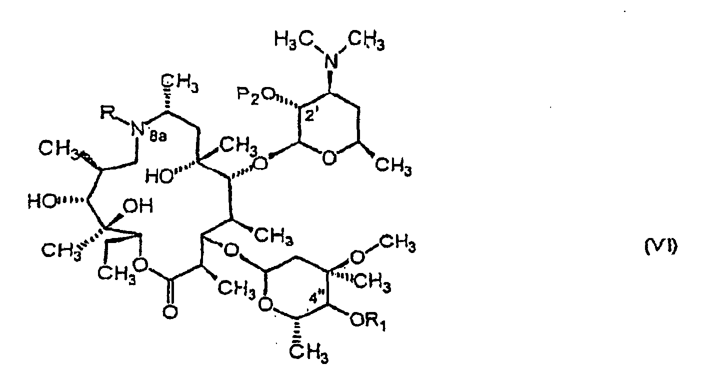 in welcher
- P₂ ein Wasserstoffatom oder eine Schutzgruppe darstellt,
- R ein Wasserstoffatom einer Alkylgruppe mit C₁ - C₁₀, einer Alkenylgruppe mit C₂ - C₁₀ oder einr Arylsulfonylgruppe mit C₆ - C₁₂, gegebenenfalls substituiert darstellt und
- OR₁ eine austretende Gruppe darstellt als Zwischenprodukt für die Darstellung einer Verbindung der allgemeinen Formel I.

18. Verbindung der allgemeinen Formel VI nach Anspruch 17, **dadurch gekennzeichnet, daß**, R eine Methylgruppe und OR₁ eine Triflatgruppe darstellt.

19. Verbindung der allgemeinen Formel VI nach Anspruch 17 oder 18, **dadurch gekennzeichnet, daß** der Kohlenstoff C-4" eine Konfiguration S besitzt.

20. Verbindung der allgemeinen Formel VII 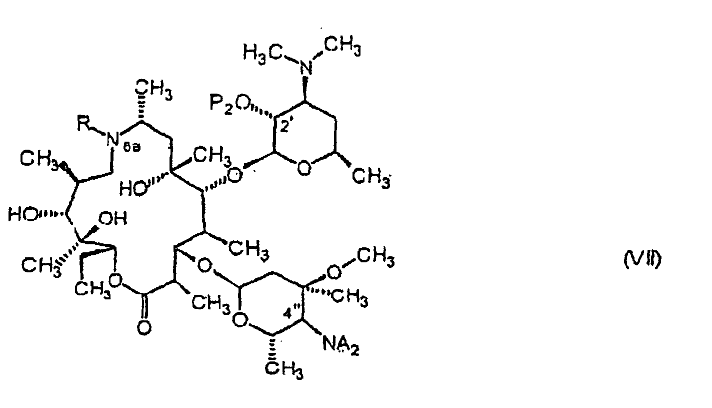 in welcher
- P₂ ein Wassersiuffatum oder eine Schutzgruppe darstellt,
- R ein Wasserstoffatom, eine Alkylgruppe mit C₁ - C₁₀, eine Alkenylgruppe mit C₂ - C₁₀ oder eine Arylsutfonylgruppe mit C₆ - C₁₂ ist, gegebenenfalls substituiert, und
- A identisch oder unterschiedlich aufweisen
• ein Stickstoffatom, gegebenenfalls substituiert,
• eine Alkylgruppe mit C₁ - C₄, substituiert durch eine oder mehrere Arylgruppen, gegebenenfalls substituiert,
als Zwischenprodukt für die Darstellung einer Verbindung der allgemeinen Formel I.

21. Verbindung der allgemeinen Formel VII nach Anspruch 20, **dadurch gekennzeichnet, daß** R eine Methylgruppe und NA₂ eine N₃-Gruppe darstellt.

22. Verbindung der allgemeinen Formel VII nach Anspruch 20 oder 21, **dadurch gekennzeichnet, daß** der Kohlenstoff C-4" eine Konfiguration R besitzt.

## Claims

1. A process for the stereoselective preparation of a compound of general formula I 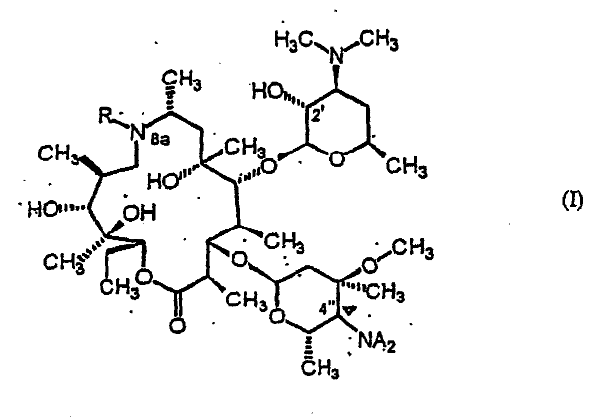 wherein:
- R is a hydrogen atom, a C₁-C₁₀ alkyl group, a C₂-C₁₀ alkenyl group, or C₆-C₁₂ arylsulfonyl group, possibly substituted, and
- A, identical or different, represent
• a hydrogen atom,
• a nitrogen atom, possibly substituted,
• a C₁-C₄ alkyl group, possibly substituted by one or more aryl groups, possibly substituted,
• a R₂CO or R₂SO₂ group with R₂ representing a hydrogen atom, a C₁-C₈ alkyl group or aryl group, possibly substituted, and
- the symbol ∇ indicates that there has been configuration inversion at the level of the C-4" carbon in relation to the compound of general formula II,
on the basis of a compound of general formula II 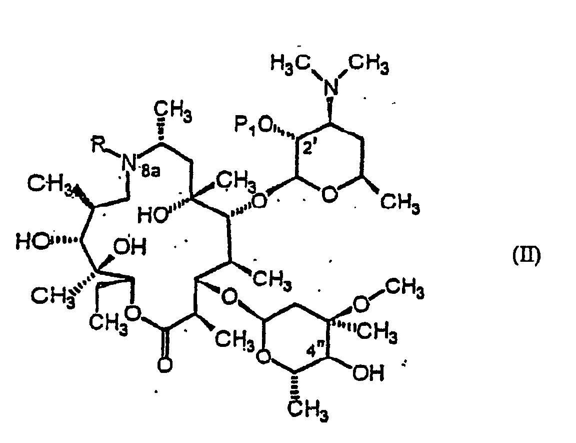
- with R as defined in general formula I, and
- P₁ representing a protector group of the hydroxyl function in position 2'
**characterised in that** it comprises at least the steps consisting in:
- activating the hydroxyl function in position 4" of the compound of general formula II in order to obtain a compound of general formula III 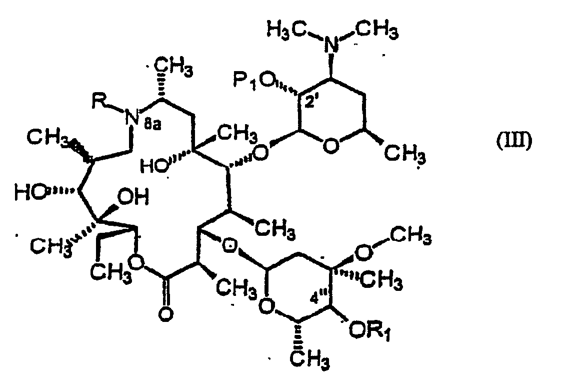 wherein:
- R and P₁ are such as defined in general formulae I and II, and
- OR₁ represents a leaving group,
- placing said compound of general formula III thus obtained in the presence of a nitrogenous nucleophilic derivative under conditions which are sufficient to permit the stereoselective displacement of the hydroxyl function activated by said nitrogenous nucleophile, and
- deprotection of the hydroxyl function in position 2',
in order to result in the expected compound of general formula I.

2. A process according to claim 1, **characterised in that** a 4"-(R)-NA₂ derivative of general formula I' is prepared 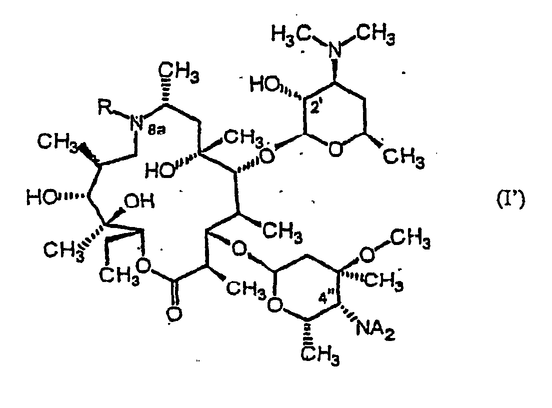 with A and R being such as defined in Claim 1
on the basis of an aza-macrolide 4"(S)-OH derivative of general formula II' 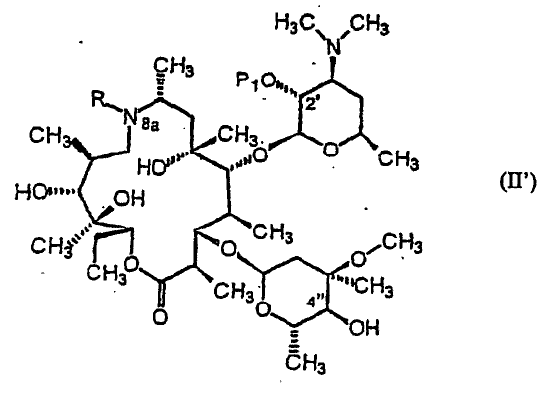 in which R and P1 are such as defined in claim 1.

3. A process according to claim 1 or claim 2, **characterised in that** the leaving group represented by OR₁ in general formula III is selected from the C₁-C₂₀ alkyl sulphonates, C₅-C₆ aryl or heteroaryl sulphonates, or C₆-C₂₆ alkylaryl sulphonates, possibly substituted by one or more halogen atoms, preferably fluorine and/or a nitro, cyano or trifluoromethyl group.

4. A process according to one of claims 1 to 3, **characterised in that** the leaving group represented by OR₁ in general formula III is a triflate group.

5. A process according to one of claims 1 to 4, **characterised in that** the leaving group derives activation from the hydroxyl function in position 4" of general formula II by a compound of formula IVA or NB 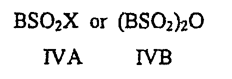 With:
- X representing a halogen atom or a nitrogenous heterocycle, preferably an imidazole cycle and
- B representing a C₁-C₂₀ alkyl sulphonate group, C₅-C₆ aryl or heteroaryl sulphonate group, or C₆-C₂₆ alkylaryl sulphonate group, possibly substituted by one or more halogen atoms, preferably fluorine and/or a nitro, cyano or trifluoromethyl group.

6. A process according to one of claims 1 to 5, **characterised in that** the nitrogenous nucleophilic compound is selected from ammonia, amines substituted or not by deprotectable groups, amides, imides, imides, sulphoneamides, sulphoneimides, hydrazines, or nitrides.

7. A process according to one of the preceding claims, **characterised in that** the nitrogenous nucleophilic compound is used at a rate of about 1 to 30 equivalents in relation to the compound of general formula III.

8. A process according to one of claims 1 to 7, **characterised in that** the nitrogenous nucleuphilic compound is an inorganic organosoluble nitride, possibly produced in situ.

9. A process according to claim 8, **characterised in that**
- the compound of general formula II is activated with a compound of general formula IVA or IVB, such as defined in claim 5,
- the compound of general formula III thus obtained is then placed in the presence of an inorganic organosoluble nitride in order to result, by stereoselective nucleophilic displacement, in a compound of general formula V 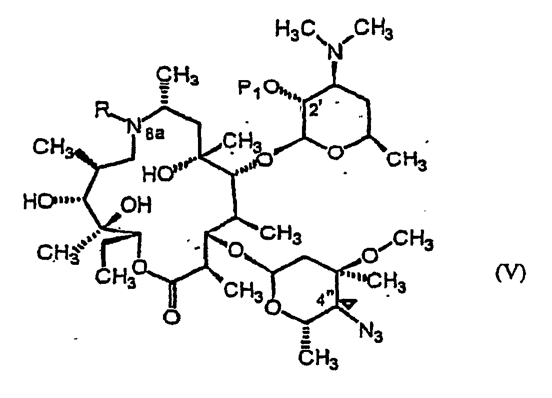
wherein R and P₁ are such as defined in general formula II, and the symbol ∇ indicates that there has been configuration inversion at the level of the C-4" carbon in relation to the compound of general formula II.

10. A process according to claim 9, **characterised in that** reduction of said compound of formula V also takes place in such a way as to obtain a compound of general formula I in which A represents a hydrogen atom.

11. A process according to claim 9 or claim 10, **characterised in that** the C-4" carbon of general formula II has an S configuration, and that of the compound of general formula V has an R configuration.

12. A process according to one of claims 8 to 11, **characterised in that** the inorganic organosoluble nitride is selected from the nitride of C₁ to C₂₀ ammonium or phosphonium tetraalkyls, substituted or unsubstituted sulphonium triaryls, and C₁ to C₂₀ guanidinum hexaalkyls.

13. A process according to one of claims 8 to 12, **characterised in that** the nitride is tetrabutylammonium nitride or tetraoctylammonium nitride.

14. A process according to one of claims 8 to 13, **characterised in that** the nucleophilic displacement of the leaving group in position 4" by an inorganic organosoluble nitride takes place within a solvent selected from aromatic solvents, such as benzene and toluene, and ethers, such as methylterbutylether and THF.

15. A process according to one of the preceding claims, **characterised in that** in the first stage activation of the hydroxyl function in position 4" takes place by a trifluoromethane sulphonate group, and nucleophilic substitution proceeds with configuration inversion with tetrabutyl ammonium nitride or tetraoctyl-ammonium nitride in toluene, at ambient temperature.

16. A process according to one of the preceding claims, **characterised in that** R represents a methyl group in general formulae I, I', I", II, II, III and V, and A represents a hydrogen atom in general formulae I and I'.

17. A compound of general formula VI 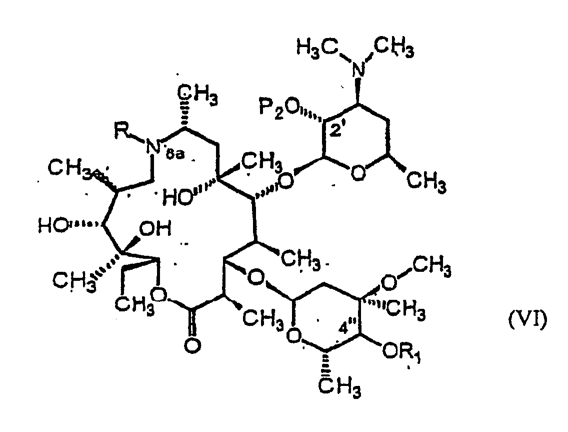 wherein:
- P₂ represents a hydrogen atom or a protector group,
- R represents a hydrogen atom, a C₁₋₁₀ alkyl group, a C₂₋₁₀ alkenyl group, or C₆-C₁₂ arylsulphonyl group, possibly substituted, and
- OR₁ represents a leaving group
by way of intermediary for the preparation of a compound of general formula I.

18. A compound of general formula VI according to claim 17, **characterised in that** R represents a methyl group, and OR₁ represents a triflate group.

19. A compound of general formula VI according to claim 17 or 18, **characterised in that** the C-4" carbon has an S configuration.

20. A compound of general formula VII wherein: 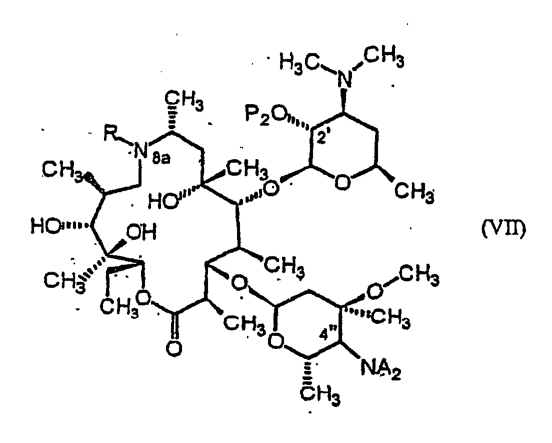
- P₂ represents a hydrogen atom or a protector group
- R is a hydrogen atom, a C₁-C₁₀ alkyl group, a C₂-C₁₀ alkenyl group, or C₆-C₁₂ arylsulphonyl group, possibly substituted, and
- A, identical or different, represent
• a nitrogen atom, possibly substituted,
• a C₁-C₄ alkyl group, substituted by one or more aryl groups, possibly substituted,
by way of intermediary for the preparation of a compound of general formula I.

21. A compound of general formula VII according to claim 20, **characterised in that** R represents a methyl group, and NA₂ represents a N₃ group.

22. A compound of general formula VII according to claim 20 or 21, **characterised in that** the C-4" carbon has an R configuration.
